(19) Europäisches Patentamt
European Patent Office
Office européen des brevets

(11) **EP 3 391 053 B1**

(12) **EUROPEAN PATENT SPECIFICATION**

(45) Date of publication and mention
of the grant of the patent:
**26.02.2020 Bulletin 2020/09**

(51) Int Cl.:
***G01N 33/574*** *(2006.01)*

(21) Application number: **16815821.0**

(86) International application number:
**PCT/EP2016/081325**

(22) Date of filing: **16.12.2016**

(87) International publication number:
**WO 2017/103034 (22.06.2017 Gazette 2017/25)**

(54) **METHODS OF DETECTING A RELAPSE OF A LUNG ADENOCARCINOMA BASED ON MARKER HUMAN EPIDIDYMIS PROTEIN 4 (HE 4) AND RELATED USES**

VERFAHREN ZUR ERKENNUNG EINES RÜCKFALLS EINES LUNGENADENOKARZINOMS AUF GRUNDLAGE DES HUMANEN EPIDIDYMISPROTEINS 4 (HE4) ALS MARKER UND ZUGEHÖRIGE VERWENDUNGEN

PROCÉDÉS DE DÉTECTION D'UNE RECHUTE DE L'ADÉNOCARCINOME DU POUMON ÉPIDIDYME SUR LA BASE DE LA PROTÉINE 4 HUMAINE (HE4) EN TANT QUE MARQUEUR ET UTILISATIONS ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2015 EP 15003607**

(43) Date of publication of application:
**24.10.2018 Bulletin 2018/43**

(73) Proprietors:
• **Roche Diagnostics GmbH**
**68305 Mannheim (DE)**
Designated Contracting States:
**DE**
• **F. Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**

(72) Inventors:
• **DAYYANI, Farshid**
**Irvine, California 92620 (US)**
• **KRAUSE, Friedemann**
**82377 Penzberg (DE)**
• **ESCHERICH, Achim**
**6052 Hergiswil (CH)**
• **WEHNL, Birgit**
**81373 München (DE)**

• **HE, Ying**
**81369 München (DE)**
• **ROLNY, Vinzent**
**80337 München (DE)**
• **RUTZ, Sandra**
**81475 München (DE)**
• **MULEY, Thomas**
**69126 Heidelberg (DE)**
• **HERTH, Felix**
**69126 Heidelberg (DE)**
• **RIEDLINGER, Julia**
**85521 Ottobrunn (DE)**

(74) Representative: **Teschemacher, Andrea**
**Patentanwälte**
**Isenbruck Bösl Hörschler PartG mbB**
**Prinzregentenstraße 68**
**81675 München (DE)**

(56) References cited:
• **SHIN-ICHI YAMASHITA ET AL: "Prognostic significance of HE4 expression in pulmonary adenocarcinoma", TUMOR BIOLOGY, vol. 32, no. 2, 15 October 2010 (2010-10-15), pages 265-271, XP055264083, CH ISSN: 1010-4283, DOI: 10.1007/s13277-010-0118-5**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KEITA TOKUISHI ET AL: "Splice variant HE4-V3 expression is associated with favorable prognosis in pulmonary adenocarcinoma", TUMOR BIOLOGY, vol. 33, no. 1, 21 October 2011 (2011-10-21), pages 103-109, XP055264088, CH ISSN: 1010-4283, DOI: 10.1007/s13277-011-0252-8
- SHIN-ICHI YAMASHITA ET AL: "Serum level of HE4 is closely associated with pulmonary adenocarcinoma progression", TUMOR BIOLOGY, vol. 33, no. 6, 22 September 2012 (2012-09-22), pages 2365-2370, XP055264090, CH ISSN: 1010-4283, DOI: 10.1007/s13277-012-0499-8
- PIERRE-JEAN LAMY ET AL: "Serum HE4: An Independent Prognostic Factor in Non-Small Cell Lung Cancer", PLOS ONE, vol. 10, no. 6, 1 June 2015 (2015-06-01), page e0128836, XP055264157, DOI: 10.1371/journal.pone.0128836
- KOTA IWAHORI ET AL: "Serum HE4 as a diagnostic and prognostic marker for lung cancer", TUMOR BIOLOGY, vol. 33, no. 4, 29 February 2012 (2012-02-29), pages 1141-1149, XP055264158, CH ISSN: 1010-4283, DOI: 10.1007/s13277-012-0356-9
- LAN W-G ET AL: "Serum human epididymis protein 4 is associated with the treatment response of concurrent chemoradiotherapy and prognosis in patients with locally advanced non-small cell lung cancer", CLINICAL AND TRANSLATIONAL ONCOLOGY, SPRINGER ITALIA SRL, ITALY, SPAIN, vol. 18, no. 4, 2 September 2015 (2015-09-02), pages 375-380, XP035637887, ISSN: 1699-048X, DOI: 10.1007/S12094-015-1375-Y [retrieved on 2015-09-02]
- J.-J. YEH ET AL: "Monitoring Cytokeratin Fragment 19 (CYFRA 21-1) Serum Levels for Early Prediction of Recurrence of Adenocarcinoma and Squamous Cell Carcinoma in the Lung after Surgical Resection", LUNG, vol. 180, no. 5, 1 October 2002 (2002-10-01), pages 273-279, XP055264103, US ISSN: 0341-2040, DOI: 10.1007/s004080000101
- C.-H. KAO ET AL: "Cytokeratin Fragment 19 (CYFRA 21-1) and Carcinoembryonic Antigen for Early Prediction of Recurrence of Lung Adenocarcinoma", LUNG, vol. 177, no. 5, 1 September 1999 (1999-09-01), pages 333-337, XP055264107, US ISSN: 0341-2040, DOI: 10.1007/PL00007651
- AKIRA MASAOKA ET AL: "Clinical Usefulness of Human Cytokeratin 19 Fragment (CYFRA 21-1) as Serum Tumor Marker for Lung Cancer", HAIGAN, vol. 34, no. 2, 1 January 1994 (1994-01-01), pages P 209-P 221, XP055264122, DOI: http://doi.org/10.2482/haigan.34.209
- FOA P ET AL: "Tumour markers CEA, NSE, SCC, TPA and CYFRA 21.1 in resectable non-small cell lung cancer", ANTICANCER RESEARCH 1999 GR, vol. 19, no. 4 C, 1999, pages 3613-3618, XP008179801, ISSN: 0250-7005
- NIKLINSKI J ET AL: "CYFRA 21-1 determination in patients with non-small cell lung cancer: Clinical utility for the detection of recurrences", JOURNAL OF CARDIOVASCULAR SURGERY, vol. 36, no. 5, 1995, pages 501-504, XP008179802, ISSN: 0021-9509

**Description**

The present invention relates to a method of detecting a relapse of a lung adenocarcinoma in an individual based on marker human epididymis protein 4 (HE4) and Cytokeratin-19 fragments (Cyfra21-1) as well as the use of the markers in the in vitro assessment of a relapse of a lung adenocarcinoma.

Lung cancer is the most common cancer worldwide, with about 1.8 million new cases and 1.6 million deaths in 2012. Treatment for lung cancer depends on the cancer's specific cell type, how far it has spread, and the person's performance status. Common treatments include palliative care, surgery, chemotherapy, and radiation therapy. Targeted therapy of lung cancer is growing in importance for advanced lung cancer. After successful therapy, patients need to be carefully monitored for a relapse. Prognostic factors in NSCLC include presence or absence of pulmonary symptoms, tumor size, cell type (histology), degree of spread (stage) and metastases to multiple lymph nodes, and vascular invasion. For NSCLC, the best prognosis is achieved with complete surgical resection of stage IA disease, with up to 70% five-year survival.

The standard of care for surveillance of patients with adenocarcinoma of the lung (ACL) after resection to detect relapse is advanced imaging, in the majority of case computed tomography. To date, in vitro tests, e.g. blood-based tests based on marker proteins, are not routinely used in the early detection of a relapse.

Cytokeratin-19 fragments (Cyfra21-1) has been suggested as a marker to detect relapse in lung cancer. It has been shown in a small number of patients with relapse (n= 15) that Cyfra21-1 levels increase before or at the time of recurrence. However, patients are not distinguished by histology, and there is also no teaching to guide clinical decision making (e.g. imaging to detect relapse) (Niklinski et al., 1995, J Cardiovasc Surg (Torino) 36(5): 501-514). This finding was confirmed by another working group, which also used Cyfra21-1, again in a small cohort of patients (n= 15), to describe increases in levels of Cyfra21-1 at relapse (Stieber et al., 1999, Anticancer Research 19: 2665-2668). However, this paper also does not distinguish between ACL (adenocarcinoma of the lung) vs non-ACL. Moreover, Cyfra21-1 has not yet become an established monitoring marker in the early diagnosis of a relapse.

Accordingly, there is still a need for a suitable in vitro testing method avoiding or minimizing the need for the above imaging methods, which are suitable only for tumors of a considerable size, which are expensive and associated with radiation exposure.

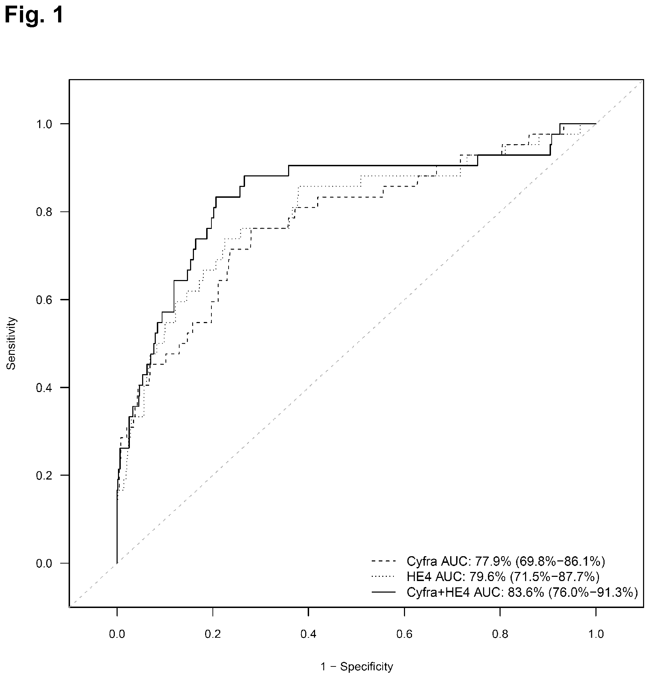

Surprisingly, it was found that human epididymis protein 4 (HE4) in combination with Cyfra21-1, is suitable in the early detection of a relapse. As shown in the Example and Figure 1, HE4 was found to be slightly superior to Cyfra21-1 and the combination of HE4 and Cyfra21-1 yielded the best results in the early detection of relapse with respect to sensitivity and area under curve (AUC).

The present disclosure provides a method of detecting a relapse of a lung adenocarcinoma in an individual, the method comprising

a) measuring in a sample obtained from the individual the amount or concentration of the marker molecule human epididymis protein 4 (HE4), and

b) detecting relapse by comparing the amount or concentration determined in step (a) to the amount or concentration as established in a control, wherein an increased value of HE4 relative to the control is indicative of the relapse.

Optionally, step (a) further comprises measuring in a sample obtained from the individual the amount or concentration of the marker molecule cytokeratin-19 fragments (CYFRA 21-1), wherein an increased value of CYFRA 21-1 relative to the control is indicative of the relapse.

In a first aspect, the present invention relates to a method of detecting a relapse of a lung adenocarcinoma in an individual, the method comprising

a) measuring in a sample obtained from the individual the amount or concentration of the marker molecules HE4 and CYFRA 21-1, and

b) detecting relapse by comparing the combined value for the markers determined in step (a) to the combined value as established in a control, wherein an increased combined value relative to the control is indicative of the relapse.

HE4 encodes for a highly conserved WAP (whey acidic protein) domain-containing protein (13kD), which is suggestive of putative serine protease inhibitor activity. In its mature glycosylated form the protein is approximately 20-25 kD, and consists of a single peptide containing two WFDC (whey acidic four-disulfide core) domains The protein is implicated in sperm maturation and potentially has a role in natural immunity, but the biological function of HE4 is unknown. Interestingly, HE4 was identified as the most upregulated gene in fibrotic kidneys of dogs. HE4 was also reported to be significantly upregulated in fibrotic kidneys of mice, and its transcript level in human kidney transplant biopsies was found to be strongly correlated with low estimated glomerular filtration rate. Despite these observations, the role of HE4 in renal fibrosis and its putative serine protease activity has remained unexplored.

The HE4 cDNA was first isolated from human epididymis (Kirchhoff et al., 1991 Biol. Reprod. 45:350-357), and HE4 cDNA was later detected with high frequency in cDNA libraries constructed from ovarian carcinomas (Wang et al., 1999 Gene 229:101; Schummer et al., 1999 Gene 238:375). The corrected sequence of HE4a was disclosed in Hellstrom et al., 2003, Cane. Res. 63:3695-3700 and in U.S. Patent 7,270,960. HE4a exhibits an amino acid sequence that is highly similar to, but distinct from, the deduced sequence of the molecule that was referred to as HE4 in earlier publications. For the purposes of this disclosure, HE4 or HE4a are considered synonymous, and are referred to synonymously herein as HE4. The amino acid sequences of HE4 and the corrected sequence HE4a are known from Kirchhoff et al., supra and US 7,270,960, respectively.

So far, HE4 has been suggested as prognostic, but not as monitoring marker. Tumor biomarkers can be divided into different types. A prognostic marker is a clinical or biologic characteristic that is objectively measurable and that provides information on the likely outcome of the cancer disease. It tells how well the patient is likely to do during the disease course (e.g. have more aggressive disease and short survival or not etc.) and how the disease will likely behave at any time-point. It aims to objectively evaluate the patient's overall outcome, such as the probability of cancer recurrence after standard treatment. Typically, prognostic biomarkers are measured and evaluated at the time of diagnosis. The presence or absence of a prognostic marker can be useful for the selection of patients for treatment. In contrast, a monitoring marker is a clinical or biologic characteristic that provides information on the time-point of cancer recurrence and predicts or indicates when cancer is to return or returned. It aims to objectively evaluate the time-point of need for a specific clinical intervention. Importantly, prognostic factors define the effects of patient or tumor characteristics on the patient outcome, whereas monitoring factors define the time-point for disease recurrence. Accordingly, the monitoring marker is measured after treatment. The measurement may be repeated in order to monitor the patient, e.g. frequently (at least 3, 4 or 5 times), at fixed intervals (e.g. every 3 or 6 months) or at predetermined points of time etc. (e.g. 3, 6 or 9 months from treatment or every full year after treatment).

Prior to the present invention, it was found that HE4 expression is associated with a worse prognosis and is a possible prognostic factor of lung adenocarcinoma. (Yamashita et al., 2011, Tumor Biol. 32: 265-271; Yamashita et al., 2012, Tumor Biol. 33: 2365-2370; Yamashita et al., 2012, J Thoracic Oncology 7(6), Suppl. 1, S44, Tokuishi et al., 2012 , Tumor Biol. 33: 103-109). Accordingly, HE4 was suggested to have a prognostic value. HE4 is not yet known as monitoring marker, still less for lung adenocarcinoma.

Cyfra21-1 belongs to the cytokeratin family. Cytokeratins are structural proteins forming the subunits of epithelial intermediary filaments, which is a major component of the cell cytoskeleton. Twenty different cytokeratin polypeptides with molecule weights ranging from 40 to 70 Kilodaltons (kD) have so far been identified. The type of cytokeratin synthesized by a cell is also affected by the growth and differentiation rate. Due to their specific distribution patterns they are eminently suitable for use as differentiation markers in tumor pathology. CYFRA21-1 is a fragment of cytokeratin 19 which is a part of cytoskeleton in epithelial cells, and can be found in an overexpressed way in tumors of epithelial origin. Intact cytokeratin polypeptides are poorly soluble, but soluble fragments can be detected in serum (Bodenmueller et al., 1994, Int. J. Biol. Markers 9: 75-81). CYFRA21-1 is a well-established marker for Non-Small-Cell Lung Carcinoma (NSCLC). The main indication for CYFRA21-1 is monitoring the course of non-small cell lung cancer (NSCLC) (Sturgeon, 2001, Clinical Chemistry 48: 1151-1159). In primary diagnosis high CYFRA21-1 serum levels indicate an advanced tumor stage and a poor prognosis in patients with non-small-cell lung cancer (van der Gaast et al., 1994, Br. J. Cancer 69: 525-528). A normal or only slightly elevated value does not rule out the presence of a tumor. Successful therapy is documented by a rapid fall in the CYFRA21-1 serum level into the normal range. A constant CYFRA21-1 value or a slight or only slow decrease in the CYFRA21-1 value indicates incomplete removal of a tumor or the presence of multiple tumors with corresponding therapeutic and prognostic consequences. CYFRA 21-1 has been suggested as monitoring marker (Yeh et al., 2002, Lung 180: 273-279 and Kao et al., 1999, Lung 177: 333-337).

Lung cancer is also known as carcinoma of the lung or pulmonary carcinoma, and is a malignant lung tumor characterized by uncontrolled cell growth in tissues of the lung. If left untreated, this growth can spread beyond the lung by process of metastasis into nearby tissue or other parts of the body. Most cancers that start in the lung, known as primary lung cancers, are carcinomas that derive from epithelial cells. The main four histological types of lung cancer are squamous cell carcinoma, adenocarcinoma, large cell carcinoma and small cell carcinoma (SCLC). The first three subtypes are generally referred to as non-small-cell carcinoma (NSCLC) and account for approximately 80% of lung cancer. Diagnosis of the lung tumor is in general based on imaging methods and analysis of biopsy samples. The 2004 World Health Organization (WHO) schema of lung tumors has been the foundation for lung cancer classification. This incorporated a number of developments, including recognition of lung carcinoma heterogeneity, the introduction of diagnostic immunohistochemical staining (IHC) techniques for the routine diagnosis of some neuroendocrine tumors, and the recognition of newly described entities such as fetal adenocarcinoma, cystic mucinous tumors, and large cell neuroendocrine carcinoma.

In 2011, a multidisciplinary expert panel representing the International Association for the Study of Lung Cancer (IASLC), the American Thoracic Society (ATS), and the European Respiratory Society (ERS) proposed a major revision of the classification system. These changes primarily affect the classification of adenocarcinoma and its distinction from squamous cell carcinoma. The present international standard for classification of tumors by oncologists and pathologists is provided by the "WHO Classification of Tumours of the Lung, Pleura, Thymus and Heart" (Travis et al, 2015, WHO Classification of Tumours, Volume 7, fourth edition). In case of doubt in the context of the present invention, the above standard is to be applied.

Adenocarcinoma is the most common type of lung cancer in contemporary series, accounting for approximately one-half of lung cancer cases. The increased incidence of adenocarcinoma is thought to be due to the introduction of low-tar filter cigarettes in the 1960s, although such causality is unproven. Lung adenocarcinoma is a type of lung cancer that forms in mucus-secreting glands throughout the body. The accepted treatment of lung adenocarcinoma stage I and II is surgical resection, with full lobar or greater resections preferable to sublobar resections (see also below). The performance of systematic mediastinal lymph node dissection improves the accuracy of staging and may have therapeutic benefits. At least 50% of these patients will develop local relapse or distant metastases. Therefore, the early detection of the relapse is of high relevance for lung adenocarcinoma.

As detailed herein, the present invention relates to detecting a relapse. A relapse is a recurrence of a past medical condition, presently the lung adenocarcinoma. The signs and symptoms of the condition return after a remission. Cancer recurrence is defined as the return of cancer after treatment and after a period of time during which the cancer cannot be detected. The same cancer reappears in the same place it was first found or very close by.

The relapse is to be detected in an individual. The individual according to the present invention may be any human or non-human animal, especially a mammal. Thus, the methods and compositions described herein are applicable to both human and veterinary disease. Evidently, non-human mammals of particular interest include domestic animals, pets, and animals of commercial value (e.g. domestic animals such as horses) or personal value (e.g. pets such as dogs, cats).

The method is especially preferred with human subjects, for which diagnostic methods including monitoring for a relapse are commonly employed. In a particularly preferred embodiment the individual is therefore a human. Alternatively or additionally, the individual is regarded as successfully treated with respect to lung adenocarcinoma, particularly by resection and/or chemotherapy. "Successfully treated" with respect to lung adenocarcinoma means that the patient after treatment does not show any medical sign and symptom of lung adenocarcinoma anymore. However, undetected cancer cells may remain in the body after treatment, causing the cancer to return later (referred to as a recurrence or relapse).

"Resection" is the surgical removal of part or all of a damaged organ or structure, particularly the removal of a tumor. Lung resection is the surgical removal of all or part of the lung. The type of resection will be based on the tumor location, size, and type, as well as your overall health and lung function prior to diagnosis. On the right side, the lung has three lobes and on the left there are two lobes. Usually, an operation for cancer involves removing a lobe, which is called a lobectomy. Wedge resection or segmentectomy refer to the removal of an area of lung smaller than a lobe, usually the tumor and a small area of healthy lung tissue around it. This is a treatment used for early-stage cancer and sometimes to remove a piece of lung where cancer is suspected but not proven. In a lobectomy, the surgeon removes a lobe of the lungs. This is the usual operation performed for lung cancer, as this has the best chance of removing all the cancerous tissue and decreasing the chance of cancer coming back. Pneumonectomy is the removal of an entire lung. This option is considered if a tumor is especially large, or in a difficult-to-reach or central position in the lung. Although pneumonectomy can result in significant loss of function, many people live quite well with only one lung.

"Chemotherapy" is a drug treatment for cancer. It is usually systemic, meaning it circulates through and affects your entire body. The drugs enter the bloodstream and kill abnormal cells or stop them from dividing. They are most often given by intravenous (IV) infusion, into a vein through a catheter, or orally. The cancer's type, stage, and location will determine the specific medicine(s), strength, and frequency of your chemotherapy. It may be combined with surgery or radiation. Chemotherapy may be applied before surgery (sometimes along with radiation therapy) to try to shrink a tumor (neoadjuvant therapy), after surgery (sometimes along with radiation therapy) to try to kill any cancer cells that may have been left behind (adjuvant therapy) or as the main treatment (sometimes along with radiation therapy) for more advanced cancers or for some people who aren't healthy enough for surgery. It is usually given in cycles, with a period of treatment (usually 1 to 3 days) followed by a rest period to allow the body time to recover. Some chemotherapeutics, though, are given every day. Cycles generally last about 3 to 4 weeks. Examples of chemotherapeutics include cisplatin, carboplatin, paclitaxel (Taxol®), albumin-bound paclitaxel (nab-paclitaxel, Abraxane®), docetaxel (Taxotere®), gemcitabine (Gemzar®), vinorelbine (Navelbine®), irinotecan (Camptosar®), etoposide (VP-16®), vinblastine or pemetrexed (Alimta®). Most often, treatment for NSCLC uses a combination of 2 chemotherapeutics. Studies have shown that adding a third drug does not add much benefit and is likely to cause more side effects. Single-drug chemo is sometimes used for people who might not tolerate combination chemotherapy well, such as those in poor overall health or who are elderly. If a combination is used, it often includes cisplatin or carboplatin plus one other drug. Sometimes combinations that do not include these drugs, such as gemcitabine with vinorelbine or paclitaxel, may be used. Individuals with advanced lung cancers who meet certain criteria, a targeted therapy drug such as bevacizumab (Avastin®) or cetuximab (Erbitux®) may be added to treatment as well.

Radiotherapy is often given together with chemotherapy, and may be used with curative intent in people with NSCLC who are not eligible for surgery. This form of high-intensity radiotherapy is called radical radiotherapy. A refinement of this technique is continuous hyperfractionated accelerated radiotherapy (CHART), in which a high dose of radiotherapy is given in a short time period. Postoperative thoracic radiotherapy generally should not be used after curative intent surgery for NSCLC. If cancer growth blocks a short section of bronchus, brachytherapy (localized radiotherapy) may be given directly inside the airway to open the passage. Compared to external beam radiotherapy, brachytherapy allows a reduction in treatment time and reduced radiation exposure to healthcare staff. Evidence for brachytherapy, however, is less than that for external beam radiotherapy.

In order to detect the relapse a sample is obtained from the individual. The sample may be any sample suitable for measuring the marker(s) according to the present invention and refers to a biological sample obtained for the purpose of evaluation in vitro. It comprises material which can be specifically related to the individual and from which specific information about the individual can be determined, calculated or inferred. A sample can be composed in whole or in part of biological material from the patient (e.g., a solid tissue sample obtained from a lung biopsy). A sample can also be material that has contacted the patient in a way that allows tests to be conducted on the sample which provides information about the individual (e.g., broncholavage fluid). The sample may preferably comprise any body fluid. Exemplary test samples include blood, serum, plasma, urine, saliva, and fluid from the lungs (such as epithelial lining fluid), e.g. obtained by bronchoscopy or broncholavage. The sample may be taken from the individual and used immediate or processed before the measuring step a). Processing may include purification (e.g. separation such as centrifugation), concentration, dilution, lysis of cellular components, freezing, acidification, conservation etc. Preferred samples are whole blood, serum, plasma or epithelial lining fluid from the lungs, with plasma, serum or whole blood representing the most convenient type of sample.

Typically, blood-related samples are preferred test samples for use in the context of the present invention. For this, blood may be drawn from a vein, usually from the inside of the elbow or the back of the hand. Particularly, in infants or young children, a sharp tool called a lancet may be used to puncture the skin and make it bleed. The blood may be collected e.g. into a pipette, or onto a slide or test strip. Accordingly, in a preferred embodiment of the present invention, the sample obtained from the individual is a blood sample, particularly selected from the group consisting of serum, plasma, and whole blood.

In the sample, the amount or concentration of the marker molecules is measured. A variety of methods for measuring a marker molecule (particularly HE4 and Cyfra21-1) are known in the art and any of these can be used.

Preferably the markers are specifically measured from a liquid sample by use of a specific binding agent.

A specific binding agent is, e.g., a receptor for the marker or an antibody to the marker or a nucleic acid complementary to the nucleic acid relating to the marker protein (e.g. a nucleic acid complementary to a marker's mRNA or relevant part thereof). Preferably, the marker molecule(s) is/are measured at the protein level.

Determination of proteins as binding partners of a marker polypeptide can be performed using any of a number of known methods for identifying and obtaining proteins that specifically interact with proteins or polypeptides, for example, a yeast two-hybrid screening system such as that described in U.S. Pat. No. 5,283,173 and U.S. Pat. No. 5,468,614, or the equivalent. A specific binding agent has preferably at least an affinity of $10^7$ l/mol for its corresponding target molecule. The specific binding agent preferably has an affinity of $10^8$ l/mol or even more preferred of $10^9$ l/mol for its target molecule. As the skilled artisan will appreciate the term specific is used to indicate that other biomolecules present in the sample do not significantly bind to the binding agent specific for the marker. Preferably, the level of binding to a biomolecule other than the target molecule results in a binding affinity which is only 10% or less, more preferably only 5% or less of the affinity to the target molecule, respectively. A preferred specific binding agent will fulfill both the above minimum criteria for affinity as well as for specificity.

A specific binding agent preferably is an antibody reactive with marker, particularly HE4 or Cyfra21-1. The term antibody refers to a polyclonal antibody, a monoclonal antibody, antigen binding fragments of such antibodies, single chain antibodies as well as to genetic constructs comprising the binding domain of an antibody.

The term "antibodies" includes polyclonal antibodies, monoclonal antibodies, fragments thereof such as F(ab')2, and Fab fragments, as well as any naturally occurring or recombinantly produced binding partners, which are molecules that specifically bind a HE4 polypeptide. Any antibody fragment retaining the above criteria of a specific binding agent can be used. Antibodies are generated by state of the art procedures, e.g., as described in Tijssen (Tijssen, P., Practice and theory of enzyme immunoassays, Elsevier Science Publishers B.V., Amsterdam (1990), the whole book, especially pages 43-78). In addition, the skilled artisan is well aware of methods based on immunosorbents that can be used for the specific isolation of antibodies. By these means the quality of polyclonal antibodies and hence their performance in immunoassays can be enhanced (Tijssen, P., *supra,* pages 108-115).

For the achievements as disclosed in the present invention polyclonal antibodies raised in e.g. goats may be used. However, clearly also polyclonal antibodies from different species, e.g., rats, rabbits or guinea pigs, as well as monoclonal antibodies can be used. Since monoclonal antibodies can be produced in any amount required with constant properties, they represent ideal tools in development of an assay for clinical routine.

For measurement the sample obtained from an individual is incubated with the specific binding agent for the marker in question under conditions appropriate for formation of a binding agent marker-complex. Such conditions need not be specified, since the skilled artisan without any inventive effort can easily identify such appropriate incubation conditions. The amount of binding agent marker-complex is measured and used in the methods and uses of the invention. As the skilled artisan will appreciate there are numerous methods to measure the amount of the specific binding agent marker-complex all described in detail in relevant textbooks (cf., e.g., Tijssen P., *supra,* or Diamandis, E.P. and Christopoulos, T.K. (eds.), Immunoassay, Academic Press, Boston (1996)).

Particularly, monoclonal antibodies to the markers (HE4 and Cyfra21-1) is used in quantitative (amount or concentration of the markers is determined) immunoassay.

Preferably, the marker is detected in a sandwich type assay format. In such assay a first specific binding agent is used to capture the marker in question on the one side and a second specific binding agent (e.g. a second antibody), which is labeled to be directly or indirectly detectable, is used on the other side. The second specific binding agent may contain a detectable reporter moiety or label such as an enzyme, dye, radionuclide, luminescent group, fluorescent group or biotin, or the like. Any reporter moiety or label could be used with the methods disclosed herein so long as the signal of such is directly related or proportional to the quantity of binding agent remaining on the support after wash. The amount of the second binding agent that remains bound to the solid support is then determined using a method appropriate for the specific detectable reporter moiety or label. For radioactive groups, scintillation counting or autoradiographic methods are generally appropriate. Antibody-enzyme conjugates can be prepared using a variety of coupling techniques (for review see, e.g., Scouten, W. H., Methods in Enzymology 135:30-65, 1987). Spectroscopic methods can be used to detect dyes (including, for example, colorimetric products of enzyme reactions), luminescent groups and fluorescent groups. Biotin can be detected using avidin or streptavidin, coupled to a different reporter group (commonly a radioactive or fluorescent group or an enzyme). Enzyme reporter groups can generally be detected by the addition of substrate (generally for a specific period of time), followed by spectroscopic, spectrophotometric or other analysis of the reaction products. Standards and standard additions can be used to determine the level of antigen in a sample, using well known techniques.

As described above, there are a variety of methods for measuring HE4. Commercially available products for measuring HE4 include an HE4 enzyme immunoassay (EIA) (Fujirebio Diagnostics Inc., Goteborg, Sweden) and the ARCHITECT HE4 assay (Abbott, Wiesbaden, Germany). Preferably, HE4 is measured using "Cobas Elecsys® HE4" (Material No: 05950929 190, Roche Diagnostics, Ltd, Rotkreuz, Switzerland), which is an electro-chemiluminescence immunoassay (ECLIA) for the quantitative determination of HE4.

Also for the measurement of Cyfra21-1 levels, there exit a variety of assay. An assay for "CYFRA21-1" specifically measures a soluble fragment of cytokeratin 19 as present in the circulation. The measurement of CYFRA21-1 is typically based upon two monoclonal antibodies (Bodenmueller et al., 1994, Int. J. Biol. Markers 9: 75-81). Commercially available products for measuring Cyfra21-1 include Enzymum-Test CYFRA 21-1 (Boehringer Mannheim, Mannheim, Germany), Cytokeratin 19 fragment (CYFRA 21-1) immunoradiometric assay kit (Cisbo Assays, Codolet, France), ELISA Kit for Cytokeratin Fragment Antigen 21-1 (Wuhan USCN Business Co., Ltd., China) and the ARCHITECT Cyfra21-1 assay (Abbott, Wiesbaden, Germany). In the CYFRA21-1 assay from Roche Diagnostics, Germany, the two specific monoclonal antibodies (KS 19.1 and BM 19.21) are used and a soluble fragment of cytokeratin 19 having a molecular weight of approx. 30,000 Daltons is measured. Preferably, CYFRA21-1 is measured on an Elecsys® analyzer using Roche product number 11820966160 according to the manufacturer's instructions.

In accordance with the present invention, the amount or concentration of a marker is determined in order to detect the relapse. The amount of a substance is a standards-defined quantity that measures the size of an ensemble of elementary entities, such as atoms, molecules, electrons, and other particles. It is sometimes referred to as chemical amount. The International System of Units (SI) defines the amount of substance to be proportional to the number of elementary entities present. The SI unit for amount of substance is the mole. It has the unit symbol mol. The concentration of a substance is the amount of a constituent divided by the total volume of a mixture. Several types of mathematical description can be distinguished: mass concentration, molar concentration, number concentration, and volume concentration. The term concentration can be applied to any kind of chemical mixture, but most frequently it refers to solutes and solvents in solutions.

The molar (amount) concentration has variants such as normal concentration and osmotic concentration.

The step of measuring the level of a marker may be carried out as follows: The sample and optionally calibrator and/or control may be contacted with the binding agent (which could be immobilized, e.g. on a solid phase) under conditions allowing the binding of the agent to the marker. Unbound binding agents may be removed by a separation step (e.g. one or more washing steps). A second agent (e.g. a labeled agent) may be added to detect the bound binding agent to allow binding to and quantification of the same. Unbound second agent may be removed. The amount of the second binding agent which is proportional to the amount of the marker may be quantified, e.g. based on the label. Quantification may done based on e.g. a calibration curve constructed for each assay by plotting measured value versus the concentration for each calibrator. The concentration or amount of marker in the sample may be then read from the calibration curve.

After the amount or concentration of the marker is determined, the value obtained is compared to the amount or concentration of the respective marker as established in a control (e.g. a control sample or control cohort or control population or a control group). The expression "comparing the amount or concentration ... to the amount or concentration as established in a control sample" is merely used to further illustrate what is obvious to the skilled artisan anyway. The control sample may be an internal or an external control. In one embodiment an internal control is used, i.e. the marker level(s) is(are) assessed in the test sample as well as in one or more other sample(s) taken from the same subject to determine if there are any changes in the level(s) of said marker(s). In another embodiment an external control is used. For an external control the presence or amount of a marker in a sample derived from the individual is compared to its amount or concentration in an individual or population of individuals known to be free of a given condition (e.g. lung cancer or relapse of lung cancer), i.e., "normal individual". Usually the sample's marker level is directly or indirectly correlated with a diagnosis and the marker level is e.g. used to determine whether an individual has or is about to have a relapse. It is within the skills of the practitioner to choose an appropriate control sample/population/cohort/group and a control or reference value for the marker established therein. It will be appreciated by the skilled artisan that such control in one embodiment is obtained from a reference population that is age-matched and free of confounding diseases. As also clear to the skilled artisan, the absolute marker values established in a control will be dependent on the assay used. Preferably samples from 100 or more well-characterized individuals from the appropriate reference population are used to establish a control (reference) value. Also preferred the reference population may be chosen to consist of at least 20, 30, 50, 200, 500 or 1000 individuals. Healthy individuals represent a preferred reference population for establishing a reference. Alternatively, reference populations may be used in methods of the invention, e.g. one population of healthy individuals and one population of individuals known to have a relapse.

In accordance with the present invention, an increased value for the amount or concentration of the marker(s) relative to the control is indicative of the relapse. If the value is increased the relapse has occurred or is about to occur. The individual identified thereby may be subject to further diagnostic or therapeutic methods, including further blood tests, imaging methods, chemotherapy or surgery. The skilled practitioner will be able to select suitable means in accordance with the medical practice of the prevailing country.

In one embodiment, the value is increased if the value amount to at least 110%, more preferably by at least 120%, more preferably by at least 130%, more preferably by at least 140%, more preferably by at least 150%, more preferably by at least 160%, more preferably by at least 170%, more preferably by at least 180%, even more preferably by at least 190% relative to the control or reference value.

Alternatively, the values for HE4 and optionally Cyfra21-1 as measured in a control group or a control population are for example used to establish a cut-off value or a reference range. A value above such cut-off value or out-side the reference range and its higher end is considered as increased. In a one embodiment a fixed cut-off value is established. Such cut-off value is chosen to match the diagnostic question of interest. In one embodiment values for HE4 and optionally Cyfra21-1 as measured in a control group or a control population are used to establish a reference range. In a preferred embodiment an HE4 and optionally Cyfra21-1 concentration is considered as increased if the value measured is above the 90%-percentile of the reference range. In further preferred embodiments a value for HE4 and optionally Cyfra21-1 is considered as increased if the value measured is above the 95%-percentile, the 96%-percentile, the 97%-percentile or the 97.5%-percentile of the reference range. In the present case, the cut-off value represents an appropriate value to distinguish an individual with relapse from an individual without relapse.

A suitable cut-off value may be chosen depending on the sensitivity and specificity desired. Sensitivity and specificity are statistical measures of the performance of a binary classification test, also known in statistics as classification function:

Sensitivity (also called the true positive rate) measures the proportion of positives that are correctly identified as such (e.g., the percentage of people with relapse who are correctly identified as having the condition).

Specificity (also called the true negative rate) measures the proportion of negatives that are correctly identified as such (e.g., the percentage of people with relapse who are correctly identified as not having the condition).

For any test, there is usually a trade-off between the measures. For instance, in an airport security setting in which one is testing for potential threats to safety, scanners may be set to trigger on low-risk items like belt buckles and keys (low specificity), in order to reduce the risk of missing objects that do pose a threat to the aircraft and those aboard (high sensitivity). This trade-off can be represented graphically as a receiver operating characteristic curve (see below). A perfect predictor would be described as 100% sensitive (e.g., all sick are identified as sick) and 100% specific (e.g., all healthy are not identified as sick); however, theoretically any predictor will possess a minimum error bound known as the Bayes error rate. The cut-off can be set in order to either increase sensitivity or specificity.

In statistics, a receiver operating characteristic (ROC), or ROC curve, is a graphical plot that illustrates the performance of a binary classifier system as its discrimination threshold is varied. The curve is created by plotting the true positive rate (TPR) against the false positive rate (FPR) at various threshold settings. As detailed above, the true-positive rate is also known as sensitivity or the sensitivity index d', known as "d-prime" in signal detection and biomedical informatics, or recall in machine learning. The false-positive rate is also known as the fall-out and can be calculated as (1 - specificity). The ROC curve compares sensitivity versus specificity across a range of values for the ability to predictor a dichotomous outcome. The area under the curve (AUC presents the overall accuracy for comparing test performance (Florkowski CM, 2008, Clin Biochem Rev 29(Suppl 1): S83-S87). Sensitivity is the ability of a test to correctly classify an individual as diseased. The ability of a test to correctly classify an individual as disease-free is called specificity. The formula is shown in Example 1 (Fawcett T, 2006, Pattern Recognition Letters 27: 861-874).

ROC analysis provides tools to select possibly optimal models and to discard suboptimal ones independently from (and prior to specifying) the cost context or the class distribution. ROC analysis is related in a direct and natural way to cost/benefit analysis of diagnostic decision making.

As two markers, namely HE4 and Cyfra21-1, are used in the methods of the invention, a combined value may be calculated using the amount/concentration of HE4 as well as the amount/concentration of Cyfra21-1. The combined value is compared to the combined value of the control sample, which has been obtained using the same mathematical procedure. In a preferred embodiment, the combined value is obtained by weighted calculation of the amount or concentration of the marker molecules in the samples. This means that one of the markers is given a higher weighting that the other. The combined value C calculated based on the level (amount or concentration) of HE4 ([HE4]) and the level (amount or concentration) of Cyfra21.1 ([Cyfra21.1]) may be obtained e.g. by the following equation:

$$\text{equation:} \qquad C = a * [HE4] + b * [Cyfra21.1],$$

wherein a and b represent the weighting factors. Preferably, the weighting factors have been obtained by analyzing a reference population.

For example, a logistic regression analysis may be performed with a binary outcome relapse and no relapse as the dependent variable and the combination of Cyfra21-1 and HE4 as the independent variables. Classification accuracy may be assessed by Area under the ROC (Receiver-Operating Characteristic) curve (AUC) and sensitivity and specificity may be calculated.

Also described is the use of HE4 as a marker molecule in the in vitro assessment of a relapse of a lung adenocarcinoma, wherein detection of an increased amount or concentration of HE4 in a sample obtained from an individual as compared to a control is indicative of the relapse.

In a second aspect, the present invention relates to the use of HE4 and CYFRA 21-1 as marker combination in the in vitro assessment of a relapse of a lung adenocarcinoma, wherein detection of an increased combined value of the marker combination in a sample obtained from an individual as compared to that of a control is indicative of the relapse.

The use according to the second aspect may be further defined as specified for the method of the first aspect of the present invention. Particularly, with respect to the terms used in the second aspect of the present disclosure it is referred to the terms, examples and specific embodiments used in the first aspect of the present disclosure, which are also applicable to the second aspect of the present disclosure.

As used herein and in the appended claims, the singular forms "a", "an", and "the" include plural reference unless the context clearly dictates otherwise. Similarly, the words "comprise", "contain" and "encompass" are to be interpreted inclusively rather than exclusively.

Unless defined otherwise, all technical and scientific terms and any acronyms used herein have the same meanings as commonly understood by one of ordinary skill in the art in the field of the disclosure.

**FIGURES**

**Figure 1** illustrates relapse detection in patients with Adeno NSCLC (relapse: 42; no relapse: 639). It shows a summary of area under curve (AUC) analysis of ROC (Receiver Operating Characteristic) curve data obtained in the experiments described herein in the Example for Cyfra21-1, HE4 and the combination of these markers.

**EXAMPLES**

**Example 1: Establishing control values and optionally weighting factors in a reference population**

Example 1 describes how control values and optionally weighting factors can be obtained in a suitable reference cohort.

An exemplary and suitable reference cohort would preferably include early stage adenocarcinoma NSCLC patients after resection but could also include healthy subjects. Healthy subjects would be treated as patients without a relapse.

Optimally for a part of the NSCLC patients a relapse was observed and concentration levels of HE4 and optionally Cyfra21-1 are known at the time of their relapse detection.

A) HE4 as only marker: determination of a cut-off value

The levels of the marker HE4 would be determined in samples taken from the reference cohort. The level of HE4 would be determined for a group of patients having a relapse and optionally for patients without relapse. A suitable cut-off value, which later can be used for assessment of new patient, can be determined as known to the skilled practitioner. The cut-off may be chosen considering suitable sensitivity and specificity of the test.

B) HE4 and Cyfra21-1 as marker combination: determination of weighting factors

The levels of the markers HE4 and Cyfra21-1 would be determined in samples taken from the reference cohort. One may use a logistic regression model to obtain the weighting factors. The dependent variable would be the binary indicator for the presence of a relapse at time point of sample collection. Independent variables would be levels of Cyfra21-1 and HE4 measured in samples taken corresponding time points.

The regression coefficients can then be used as weighting factors to calculate the combined value C for each patient in the reference cohort.

Based on this combined value C one now may establish a reference value which later can be used for assessment of new patient. Depending on the desired proportion such a reference value should have one would establish the reference value on patients with a detected relapse or on patients without a relapse.

If one would like to have a high certainty to detect a relapse in a new patient one would choose a reference value in a way that the combined values C of the majority of the patients with a detected relapse lay above this reference value (e.g. 90% of the patients).

If one however would like to have a high certainty that a new patient for which the combined value is above the reference value has really a relapse and is not a false positive one would choose the reference value in such a way that the majority (e.g. 90%) of patients without a relapse have a smaller combined value C as the reference value.

**Example 2: Biomarker multivariate analysis in a cohort of NSCLC patient**

The analysis was aim to identify the relapsed patients based on serum biomarker levels of Cyfra21-1 and HE4.

The analysis population included 130 early stage adenocarcinoma NSCLC patients which all underwent a resection of the tumor. Patients were periodically seen and at each visit it was recorded if a relapse occurred and blood samples were taken and concentrations of Cyfra and HE4 were measured. In total 681 visits were recorded. In 42 out of these 681 visits a relapse was detected.

A logistic regression analysis with binary outcome relapse and no relapse at each visit as the dependent variable and the combination of Cyfra21-1 and HE4 levels at the corresponding visits as the independent variables was established as described in Example 1.

Based on the coefficients derived from the logistic regression model a combined value C was calculated for each patient. The formula of C in this example was:

$$C = -10.472 + \ln(\text{Cyfra21-1}) * 1.424 + \ln(\text{HE4}) * 1.525$$

where ln() is the natural logarithm.

Subsequently, it was evaluated how well this combined value C can discriminate between visits with or without a relapse. Classification accuracy was assessed by Area under the ROC (Receiver-Operating Characteristic) curve (AUC) as well as sensitivity and specificity was reported.

ROC (Receiver-Operating Characteristic) curve is a graphical method that illustrates the performance of a binary classifier system as its discrimination threshold is varied. It compares sensitivity versus specificity across a range of values for the ability to predictor a dichotomous outcome. AUC presents the overall accuracy for comparing test performance (Christopher M F 2008 Clin Biochem Rev). Sensitivity is the ability of a test to correctly classify an individual as diseased. The ability of a test to correctly classify an individual as disease-free is called specificity. The formula is shown below (Fawcett Tom 2006 Pattern Recognition Letters).

|  | Disease present | Disease absent | Total |
|---|---|---|---|
| Test positive | True positive (TP) | False positive (FP) | TP+FP |
| Test negative | False negative (FN) | True negative (TN) | FN+TN |
| Total | TP+FN | TN+FP | |

$$\text{Sensitivity} = TP/(TP+FN)$$
$$\text{Specificity} = TN/(TN+FP)$$

Cyfra21-1:

AUC: 77.9 %
Sensitivity (@90.0% Spec): 45.2%
Specificity (@88.1% Sens): 33.3%

HE4:

AUC: 79.6 %
Sensitivity (@90.0% Spec): 52.4%, Cutoff: 113.1 pmol/mL
Specificity (@88.1% Sens): 28.3%, Cutoff: 58.8 pmol/mL

Cyfra + HE4:

AUC: 83.6 %
Sensitivity (@90.1% Spec): 57.1%, Cutoff Value on C scale: -2.279
Specificity (@88.1% Sens): 64.2%, Cutoff Value on C scale: - 3.203

**Example 3: Monitoring of new patient**

If one would wish to use HE4 and optionally Cyfra21-1 for monitoring of a new patient after resection one would collect blood samples periodically at suitable time points and measure the concentration of HE4 and optionally Cyfra21-1 in each sample. For each sample/visit one would then calculate the combined value C with the use of the weighting factors which could be established as described in Example 1 or one could use these from Example 2. If this combined value C in a newly taken blood sample is higher than the reference value then one would assume that this patient has a relapse. The reference value could be established as described in Example 1 or be taken from Example 2.

**Claims**

1. A method of detecting a relapse of a lung adenocarcinoma in an individual, the method comprising

   a) measuring in a sample obtained from the individual the amount or concentration of the marker molecules HE4 and CYFRA 21-1, and
   b) detecting relapse by comparing the combined value for the markers determined in step (a) to the combined value as established in a control, wherein an increased combined value relative to the control is indicative of the relapse.

2. The method of claim 1, wherein the combined value is obtained by weighted calculation of the amount or concentration of the marker molecules in the samples.

3. The method of claim 2, wherein the weighting factors have been obtained by analyzing a reference population.

4. The method of any of claims 1 to 3, wherein the sample is a blood sample, particularly selected from the group consisting of serum, plasma, and whole blood.

**5.** The method of any of claims 1 to 4, wherein the marker molecule(s) is/are measured at the protein level.

**6.** The method of any of claims 1 to 5, wherein the individual is a human.

**7.** The method of any of claims 1 to 6, wherein the individual is regarded as successfully treated with respect to lung adenocarcinoma, particularly by resection and/or chemotherapy.

**8.** Use of HE4 and CYFRA 21-1 as marker combination in the in vitro assessment of a relapse of a lung adenocarcinoma, wherein detection of an increased combined value of the marker combination in a sample obtained from an individual as compared to that of a control is indicative of the relapse.

**9.** The use of claim 8, wherein the use is further defined as specified in any of claims 2 to 7.


**Patentansprüche**

**1.** Verfahren zum Nachweisen eines Rückfalls in einem Individuum mit Lungenadenokarzinom, wobei das Verfahren umfasst

a) Messen der Menge oder Konzentration der Markermoleküle HE4 und CYFRA 21-1 in einer von dem Individuum erhaltenen Probe und
b) Nachweisen des Rückfalls durch Vergleichen des kombinierten Wertes für die in Schritt (a) bestimmten Marker mit dem kombinierten Wert, wie er in einer Kontrolle bestimmt wurde, wobei ein erhöhter kombinierter Wert im Vergleich zur Kontrolle für den Rückfall indikativ ist.

**2.** Verfahren nach Anspruch 1, wobei der kombinierte Wert durch gewichtete Berechnung der Menge oder Konzentration der Markermoleküle in den Proben erhalten wird.

**3.** Verfahren nach Anspruch 2, wobei die Gewichtungsfaktoren durch Analysieren einer Referenzpopulation erhalten wurden.

**4.** Verfahren nach einem der Ansprüche 1 bis 3, wobei die Probe eine Blutprobe ist, insbesondere ausgewählt aus der Gruppe bestehend aus Serum, Plasma und Vollblut.

**5.** Verfahren nach einem der Ansprüche 1 bis 4, wobei das/die Markermolekül(e) auf Proteinebene gemessen wird/werden.

**6.** Verfahren nach einem der Ansprüche 1 bis 5, wobei das Individuum ein Mensch ist.

**7.** Verfahren nach einem der Ansprüche 1 bis 6, wobei das Individuum als erfolgreich in Bezug auf das Lungenadenokarzinom behandelt gilt, insbesondere durch Resektion und/oder Chemotherapie.

**8.** Verwendung von HE4 und CYFRA 21-1 als Markerkombination bei der in vitro-Beurteilung eines Rückfalls bei einem Lungenadenokarzinom, wobei der Nachweis eines erhöhten kombinierten Wertes der Markerkombination in einer Probe, die von einem Individuum erhalten wurde, im Vergleich zu dem einer Kontrolle, indikativ für den Rückfall ist.

**9.** Verwendung nach Anspruch 8, wobei die Verwendung weiter definiert ist, wie in einem der Ansprüche 2 bis 7 angegeben.


**Revendications**

**1.** Procédé de détection d'une récidive d'un adénocarcinome pulmonaire chez un individu, le procédé comprenant le fait de :

a) mesurer, dans un échantillon obtenu de l'individu, la quantité ou la concentration des molécules marqueurs HE4 et CYFRA 21-1 ; et
b) détecter une récidive en comparant la valeur combinée pour les marqueurs déterminés à l'étape (a) à la valeur combinée telle qu'établie dans un témoin, dans lequel une augmentation de la valeur combinée par rapport à celle du témoin est révélatrice de la récidive.

2.  Procédé selon la revendication 1, dans lequel on obtient la valeur combinée via un calcul pondéré de la quantité ou de la concentration des molécules marqueurs dans les échantillons.

3.  Procédé selon la revendication 2, dans lequel les facteurs de pondération ont été obtenus par analyse d'une population de référence.

4.  Procédé selon l'une quelconque des revendications 1 à 3, dans lequel l'échantillon représente un échantillon de sang, en particulier choisi parmi le groupe constitué par du sérum, du plasma et du sang total.

5.  Procédé selon l'une quelconque des revendications 1 à 4, dans lequel la/les molécule(s) marqueur(s) est/sont mesurée(s) au niveau protéique.

6.  Procédé selon l'une quelconque des revendications 1 à 5, dans lequel l'individu est un être humain.

7.  Procédé selon l'une quelconque des revendications 1 à 6, dans lequel l'individu est considéré comme ayant subi un traitement couronné de succès par rapport à un adénocarcinome pulmonaire, en particulier par résection et/ou par chimiothérapie.

8.  Utilisation de HE4 et de CYFRA 21-1 à titre d'une combinaison de marqueurs dans l'évaluation in vitro d'une récidive d'un adénocarcinome pulmonaire, dans laquelle la détection d'une augmentation de la valeur combinée de la combinaison de marqueurs dans un échantillon obtenu d'un individu telle qu'on la compare à celle d'un témoin est révélatrice de la récidive.

9.  Utilisation selon la revendication 8, dans laquelle l'utilisation est en outre définie comme spécifiée dans l'une quelconque des revendications 2 à 7.

# Fig. 1

## REFERENCES CITED IN THE DESCRIPTION

### Patent documents cited in the description

- US 7270960 B
- US 5283173 A
- US 5468614 A

### Non-patent literature cited in the description

- NIKLINSKI et al. *J Cardiovasc Surg (Torino),* 1995, vol. 36 (5), 501-514
- STIEBER et al. *Anticancer Research,* 1999, vol. 19, 2665-2668
- KIRCHHOFF et al. *Biol. Reprod.,* 1991, vol. 45, 350-357
- WANG et al. *Gene,* 1999, vol. 229, 101
- SCHUMMER et al. *Gene,* 1999, vol. 238, 375
- HELLSTROM et al. *Cane. Res.,* 2003, vol. 63, 3695-3700
- YAMASHITA et al. *Tumor Biol.,* 2011, vol. 32, 265-271
- YAMASHITA et al. *Tumor Biol.,* 2012, vol. 33, 2365-2370
- YAMASHITA et al. *J Thoracic Oncology,* 2012, vol. 7 (6), 44
- TOKUISHI et al. *Tumor Biol.,* 2012, vol. 33, 103-109
- BODENMUELLER et al. *Int. J. Biol. Markers,* 1994, vol. 9, 75-81
- STURGEON. *Clinical Chemistry,* 2001, vol. 48, 1151-1159
- VAN DER GAAST et al. *Br. J. Cancer,* 1994, vol. 69, 525-528
- YEH et al. *Lung,* 2002, vol. 180, 273-279
- KAO et al. *Lung,* 1999, vol. 177, 333-337
- WHO Classification of Tumours of the Lung, Pleura, Thymus and Heart. TRAVIS et al. WHO Classification of Tumours. 2015, vol. 7
- TIJSSEN, P. Practice and theory of enzyme immunoassays. Elsevier Science Publishers B.V, 1990
- Immunoassay. Academic Press, 1996
- SCOUTEN, W. H. *Methods in Enzymology,* 1987, vol. 135, 30-65
- FLORKOWSKI CM. *Clin Biochem Rev,* 2008, vol. 29 (1), S83-S87
- FAWCETT T. *Pattern Recognition Letters,* 2006, vol. 27, 861-874
- CHRISTOPHER M F. *Clin Biochem Rev,* 2008
- FAWCETT TOM. *Pattern Recognition Letters,* 2006